# FASCICULE DE BREVET EUROPEEN

(11) **EP 1 863 871 B1**
(45) Date de publication et mention de la délivrance du brevet: **12.08.2009**
(21) Numéro de dépôt: 06723456.7
(22) Date de dépôt: 15.03.2006
(51) Int. Cl.: C08K 5/3415, B60C 1/00, C07D 207/44, C07D 403/10

(54) **COMPOSITION DE CAOUTCHOUC COMPORTANT UN ITACONIMIDOMALEIMIDE**
KAUTSCHUKZUSAMMENSETZUNG, ENTHALTEND EIN ITACONIMIDOMALEINIMID
RUBBER COMPOSITION COMPRISING AN ITACONIMIDO-MALEIMIDE

(30) Priorité: 24.03.2005 FR 0502916
(43) Date de publication de la demande: 12.12.2007
(73) Titulaire: Société de Technologie Michelin, 63000 Clermont-Ferrand (FR); Michelin Recherche et Technique S.A., 1763 Granges-Paccot (CH)
(72) Inventeur: BELIN, Laure, F-63200 Riom (FR); GANDON-PAIN, Sylvie, 63100 Clermont-Ferrand (FR); ARAUJO DA SILVA, José Carlos, 63430 Pont du Château (FR); LAFFARGUE, Géraldine, 63100 Clermont-Ferrand (FR)
(74) Mandataire: Cohen, Sylvia
(86) Numéro de dépôt international: PCT/EP2006/002391
(87) Numéro de publication internationale: WO 2006/099985

(56) Documents cités:
- EP-A- 0 345 825
- WO-A-92/07904
- FR-A- 2 859 730

## Description

La présente invention se rapporte aux compositions de caoutchouc destinées notamment à la fabrication de pneumatiques ou de produits semi-finis pour pneumatiques, ainsi qu'aux agents antiréversion utilisables pour la protection thermique de telles compositions.

Depuis la découverte de la vulcanisation ou réticulation du caoutchouc par le soufre, de nombreuses améliorations ont été apportées au procédé de base, mais le soufre reste encore de nos jours l'élément indispensable d'un point de vue industriel à la réticulation des élastomères diéniques.

Le principe de la vulcanisation réside dans la création de ponts de soufre entre deux macromolécules par réaction sur les doubles liaisons de ces élastomères diéniques. Une des caractéristiques remarquables de la vulcanisation est la simplicité avec laquelle cette réaction peut être contrôlée par ajout de composés présentant un effet accélérateur ou retardateur. En jouant sur les taux respectifs de soufre et d'accélérateurs, il est notamment possible de contrôler le rendement de vulcanisation, d'obtenir des ponts de soufre de différentes configurations qui conduisent, pour une composition de caoutchouc donnée, à des ajustements possibles des propriétés, tant à l'état cru qu'à l'état cuit.

Toutefois, la vulcanisation au soufre a pour inconvénient connu de conduire à une résistance limitée des vulcanisats obtenus, due au vieillissement thermique de ces derniers ("thermal ageing"). En particulier, les vulcanisats d'élastomères diéniques réticulés à partir du soufre présentent une sensibilité importante à la température lorsque cette dernière atteint une valeur voisine de la température de cuisson ou vulcanisation initiale. Il s'ensuit une chute de la densité des ponts de soufre formés initialement lors de la vulcanisation, la distribution du réseau de vulcanisation évoluant vers un raccourcissement, c'est-à-dire une diminution des ponts polysulfures au profit des ponts monosulfures. Ce phénomène, connu sous le terme de réversion, s'accompagne d'une dégradation des propriétés mécaniques du vulcanisat.

Aussi a-t-on tenté de supprimer, tout au moins limiter ce phénomène de réversion en utilisant dans des compositions de caoutchouc des additifs spécifiques, appelés agents antiréversion, permettant de stabiliser thermiquement les vulcanisats. Ces agents antiréversion constituent aujourd'hui un pôle de recherche important, particulièrement dans le domaine du pneumatique pour lequel une stabilité thermique optimale est recherchée.

Une famille largement décrite d'agents antiréversion est constituée par les composés maléimide, plus particulièrement par les bismaléimides utilisés seuls ou en association avec d'autres composés (voir par exemple EP 191931 ou US 4803250, EP 640114 ou WO93/23467, EP 703943 ou US 5872188, EP 709234 ou US 5503940, EP 823453 ou US 6079468, EP 988999, US 5328636, US 5616279, US 5623007, WO92/07904 ou US 5426155, WO95/16738, demande JP2001-226528).

Les Demanderesses ont découvert lors de leurs recherches que, de manière inattendue, certains composés maléimide bien spécifiques permettent d'offrir aux vulcanisats une résistance à la réversion améliorée comparativement aux bismaléimides précitées. Ces composés ne nécessitent en outre la présence d'aucun coagent pour réduire de manière significative le niveau de réversion des vulcanisats.

En conséquence, un premier objet de l'invention concerne une composition de caoutchouc utilisable pour la fabrication de pneumatiques, à base d'au moins (i) un élastomère diénique, (ii) une charge renforçante, (iii) un système de vulcanisation et (iv) un composé maléimide, caractérisé en ce que ledit composé maléimide est un itaconimidomaléimide de formule spécifique (R radical hydrocarboné comportant de 1 à 25 atomes de carbone et éventuellement un ou plusieurs hétéroatome(s) choisi(s) parmi O, N et S) :

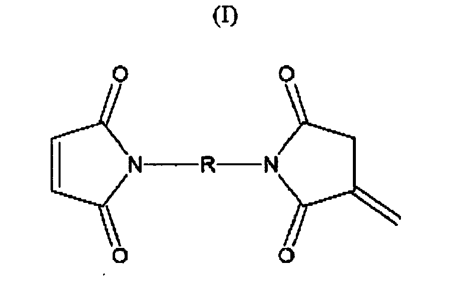

A la connaissance des Demanderesses, un tel composé itaconimidomaléimide n'avait jusqu'ici jamais été utilisé dans une composition de caoutchouc utilisable notamment pour la fabrication de pneumatiques

L'invention a également pour objet un procédé pour préparer une composition de caoutchouc utilisable pour la fabrication de pneumatiques et présentant une résistance à la réversion améliorée, cette composition étant à base d'un élastomère diénique, d'une charge renforçante et d'un système de vulcanisation, ledit procédé comportant les étapes suivantes :
- incorporer à un élastomère diénique, au cours d'une première étape dite "non-productive", au moins une charge renforçante, en malaxant thermomécaniquement le tout, en une ou plusieurs fois, jusqu'à atteindre une température maximale comprise entre 110°C et 190°C ;
- refroidir l'ensemble à une température inférieure à 100°C ;
- incorporer ensuite, au cours d'une seconde étape dite "productive", le système de vulcanisation ;
- malaxer le tout jusqu'à une température maximale inférieure à 110°C,
et étant caractérisé en ce qu'est en outre incorporé, au cours de l'une quelconque des étapes du procédé, un composé itaconimidomaléimide de formule (I).

L'invention a également pour objet l'utilisation d'une composition selon l'invention pour la fabrication d'un article fini ou d'un produit semi-fini en caoutchouc destiné à tout système de liaison au sol des véhicules automobiles, tel que pneumatique, appui interne de sécurité pour pneumatique, roue, ressort en caoutchouc, articulation élastomérique, autre élément de suspension et anti-vibratoire.

L'invention a tout particulièrement pour objet l'utilisation d'une composition selon l'invention pour la fabrication de pneumatiques ou de produits semi-finis en caoutchouc destinés à ces pneumatiques, ces produits semi-finis étant de préférence choisis dans le groupe constitué par les bandes de roulement, les nappes d'armature de sommet, les flancs, les nappes d'armature de carcasse, les bourrelets, les protecteurs, les sous-couches, les blocs de caoutchouc et autres gommes internes, notamment les gommes de découplage, destinés à assurer la liaison ou l'interface entre les zones précitées des pneumatiques.

L'invention a également pour objet les articles finis et produits semi-finis en caoutchouc eux-mêmes, en particulier les pneumatiques et produits semi-finis pour pneumatiques, lorsqu'ils comportent une composition élastomérique conforme à l'invention. Les pneumatiques conformes à l'invention sont notamment destinés à des véhicules tourisme comme à des véhicules industriels choisis parmi camionnettes, "Poids-lourd" - i.e., métro, bus, engins de transport routier (camions, tracteurs, remorques), véhicules hors-la-route - , engins agricoles ou de génie civil, avions, autres véhicules de transport ou de manutention.

L'invention ainsi que ses avantages seront aisément compris à la lumière de la description et des exemples de réalisation qui suivent, ainsi que des figures annexées qui schématisent des procédés d'obtention de composés itaconimidomaléimide utilisables dans les compositions de l'invention.

### I. MESURES ET TESTS UTILISES

Les compositions de caoutchouc sont caractérisées avant et après cuisson, comme indiqué ci-après.

### I-1. Rhéométrie

Les mesures sont effectuées à 150°C avec un rhéomètre à chambre oscillante, selon la norme DIN 53529 - partie 3 (juin 1983). L'évolution du couple rhéométrique en fonction du temps décrit l'évolution de la rigidification de la composition par suite de la réaction de vulcanisation. Les mesures sont traitées selon la norme DIN 53529 - partie 2 (mars 1983) : les couples minimum et maximum, mesurés en dN.m (déciNewton.mètre), sont respectivement nommés Cₘᵢₙ et Cₘₐₓ ; on mesure également l'écart noté ΔCouple (en dN.m) entre Cₘₐₓ et Cₘᵢₙ qui permet d'apprécier le rendement de vulcanisation.

Sauf indication contraire, les propriétés mécaniques indiquées ci-après (paragraphe I-4) sont celles mesurées à « optimum de cuisson », c'est-à-dire, de manière connue, celles obtenues, pour une température de cuisson déterminée, après la durée de cuisson minimale pour atteindre le couple rhéométrique maximum Cₘₐₓ.

### I-2. Essais de traction

Ces essais permettent de déterminer les contraintes d'élasticité et les propriétés à la rupture. Sauf indication différente, ils sont effectués conformément à la norme française NF T 46-002 de septembre 1988. On mesure en seconde élongation (i.e. après un cycle d'accommodation au taux d'extension prévu pour la mesure elle-même) les modules sécants nominaux (ou contraintes apparentes, en Mpa) à 10% d'allongement (noté MA10), 100% d'allongement (noté MA100) et 300% d'allongement (noté MA300). Toutes ces mesures de traction sont effectuées dans les conditions normales de température (23 ± 2°C) et d'hygrométrie (50 ± 5% d'humidité relative), selon la norme française NF T 40-101 (décembre 1979).

### I-3. Mesure de la réversion

La réversion peut être analysée selon différentes méthodes, le but étant de déterminer, de manière indirecte, l'évolution de la densité des ponts de soufre, entre une cuisson dite à l'optimum (correspondant au couple maximum Cₘₐₓ) et une cuisson prolongée.

La première approche consiste à mesurer l'évolution (diminution) du couple rhéométrique : le paramètre ΔR₆₀ représente l'évolution en % du couple entre Cₘₐₓ et le couple mesuré après 60 min de cuisson, à une température de cuisson déterminée (par exemple 150°C). Plus le paramètre ΔR₆₀ est élevé, plus le phénomène de réversion est important.

La seconde approche consiste à mesurer l'évolution (diminution) des modules MA100 ou MA300 précités : les paramètres ΔMA100 et ΔMA300 correspondent à l'évolution en % des modules respectifs mesurés à l'optimum de cuisson (Cₘₐₓ) et après une cuisson de 6 heures, à une température de cuisson déterminée (150°C). Plus les paramètres ΔMA100 ou ΔMA300 sont élevés, plus le phénomène de réversion est important.

### II. CONDITIONS DE REALISATION DE L'INVENTION

Les compositions de caoutchouc selon l'invention sont à base d'au moins un (c'est-à-dire un ou plusieurs ) élastomère diénique(s), une (une ou plusieurs) charge(s) renforçante(s), un (un ou plusieurs) système(s) de réticulation et un (un ou plusieurs) composé(s) itaconimidomaléimide de formule (I) précitée.

Bien entendu, par l'expression composition « à base de », il faut entendre une composition comportant le mélange et/ou le produit de réaction des différents constituants utilisés, certains de ces constituants de base étant susceptibles de, ou destinés à réagir entre eux, au moins en partie, lors des différentes phases de fabrication de la composition, en particulier au cours de sa vulcanisation.

Dans la présente description, sauf indication expresse différente, tous les pourcentages (%) indiqués sont des % en masse.

### II-1. Elastomère diénique

Par élastomère ou caoutchouc « diénique », on entend de manière connue un élastomère issu au moins en partie (i.e. un homopolymère ou un copolymère) de monomères diènes (monomères porteurs de deux doubles liaisons carbone-carbone, conjuguées ou non).

Ces élastomères diéniques peuvent être classés dans deux catégories : « essentiellement insaturés » ou « essentiellement saturés ».

On entend en général par « essentiellement insaturé », un élastomère diénique issu au moins en partie de monomères diènes conjugués, ayant un taux de motifs ou unités d'origine diénique (diènes conjugués) qui est supérieur à 15% (% en moles).

C'est ainsi, par exemple, que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques « essentiellement saturés » (taux de motifs d'origine diénique faible ou très faible, toujours inférieur à 15%).

Dans la catégorie des élastomères diéniques « essentiellement insaturés », on entend en particulier par élastomère diénique « fortement insaturé » un élastomère-diénique ayant un taux de motifs d'origine diénique (diènes conjugués) qui est supérieur à 50%.

Ces définitions étant données, on entend plus particulièrement par élastomère diénique susceptible d'être mis en oeuvre dans les compositions conformes à l'invention :
(a) - tout homopolymère obtenu par polymérisation d'un monomère diène conjugué ayant de 4 à 12 atomes de carbone ;
(b) - tout copolymère obtenu par copolymérisation d'un ou plusieurs diènes conjugués entre eux ou avec un ou plusieurs composés vinyle aromatique ayant de 8 à 20 atomes de carbone ;
au-delà - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène ;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, l'homme du métier du pneumatique comprendra que la présente invention est en premier lieu mise en oeuvre avec des élastomères diéniques essentiellement insaturés, en particulier du type (a) ou (b) ci-dessus.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène. A titre de composés vinyle-aromatiques conviennent par exemple le styrène, l'ortho-, méta-, paraméthylstyrène, le mélange commercial « vinyle-toluène », le para-tertiobutylstyrène, les méthoxystyrènes, les chlorostyrènes, le vinylmésitylène, le divinylbenzène, le vinylnaphtalène.

Les copolymères peuvent contenir entre 99% et 20% en poids d'unités diéniques et entre 1% et 80% en poids d'unités vinyle-aromatiques. Les élastomères peuvent avoir toute microstructure qui est fonction des conditions de polymérisation utilisées, notamment de la présence ou non d'un agent modifiant et/ou randomisant et des quantités d'agent modifiant et/ou randomisant employées. Les élastomères peuvent être par exemple à blocs, statistiques, séquencés, microséquencés, et être préparés en dispersion ou en solution ; ils peuvent être couplés et/ou étoilés ou encore fonctionnalisés avec un agent de couplage et/ou d'étoilage ou de fonctionnalisation.

Conviennent les polybutadiènes et en particulier ceux ayant une teneur en unités -1,2 comprise entre 4% et 80% ou ceux ayant une teneur en cis-1,4 supérieure à 80%, les polyisoprènes, les copolymères de butadiène-styrène et en particulier ceux ayant une teneur en styrène comprise entre 5% et 50% en poids et plus particulièrement entre 20% et 40%, une teneur en liaisons -1,2 de la partie butadiénique comprise entre 4% et 65%, une teneur en liaisons trans-1,4 comprise entre 20% et 80%, les copolymères de butadiène-isoprène et notamment ceux ayant une teneur en isoprène comprise entre 5% et 90% en poids et une température de transition vitreuse (Tg, mesurée selon ASTM D3418-82) de -40°C à -80°C, les copolymères isoprène-styrène et notamment ceux ayant une teneur en styrène comprise entre 5% et 50% en poids et une Tg comprise entre -25°C et -50°C. Dans le cas des copolymères de butadiène-styrène-isoprène conviennent notamment ceux ayant une teneur en styrène comprise entre 5% et 50% en poids et plus particulièrement comprise entre 10% et 40%, une teneur en isoprène comprise entre 15% et 60% en poids et plus particulièrement entre 20% et 50%, une teneur en butadiène comprise entre 5% et 50% en poids et plus particulièrement comprise entre 20% et 40%, une teneur en unités -1,2 de la partie butadiénique comprise entre 4% et 85%, une teneur en unités trans -1,4 de la partie butadiénique comprise entre 6% et 80%, une teneur en unités -1,2 plus -3,4 de la partie isoprénique comprise entre 5% et 70% et une teneur en unités trans -1,4 de la partie isoprénique comprise entre 10% et 50%, et plus généralement tout copolymère butadiène-styrène-isoprène ayant une Tg comprise entre -20°C et -70°C.

En résumé, l'élastomère diénique de la composition conforme à l'invention est choisi préférentiellement dans le groupe des élastomères diéniques fortement insaturés constitué par les polybutadiènes (BR), les polyisoprènes (IR), le caoutchouc naturel (NR), les copolymères de butadiène, les copolymères d'isoprène et les mélanges de ces élastomères. De tels copolymères sont plus préférentiellement choisis dans le groupe constitué par les copolymères de butadiène-styrène (SBR), les copolymères d'isoprène-butadiène (BIR), les copolymères d'isoprène-styrène (SIR) et les copolymères d'isoprène-butadiène-styrène (SBIR).

Selon un mode de réalisation particulièrement préférentiel, l'élastomère diénique est majoritairement (c'est-à-dire pour plus de 50 pce) un SBR, qu'il s'agisse d'un SBR préparé en émulsion (« ESBR ») ou d'un SBR préparé en solution (« SSBR »), ou un coupage (mélange) SBR/BR, SBR/NR (ou SBR/IR), ou encore BR/NR (ou BR/IR). Dans le cas d'un élastomère SBR, on utilise notamment un SBR ayant une teneur en styrène comprise entre 20% et 30% en poids, une teneur en liaisons vinyliques de la partie butadiénique comprise entre 15% et 65%, une teneur en liaisons trans-1,4 comprise entre 15% et 75% et une Tg comprise entre -20°C et -55°C; un tel SBR peut être avantageusement utilisé en mélange avec un BR possédant de préférence plus de 90% de liaisons cis-1,4.

Selon un autre mode de réalisation particulièrement préférentiel, l'élastomère diénique est majoritairement (pour plus de 50 pce) un élastomère isoprénique. C'est le cas en particulier lorsque les compositions de l'invention sont destinées à constituer, dans les pneumatiques, les matrices de caoutchouc de certaines bandes de roulement (par exemple pour véhicules industriels), de nappes d'armature de sommet (par exemple de nappes de travail, nappes de protection ou nappes de frettage), de nappes d'armature de carcasse, de flancs, de bourrelets, de protecteurs, de sous-couches, de blocs de caoutchouc et autres gommes internes assurant l'interface entre les zones précitées des pneumatiques.

Les compositions selon l'invention sont par exemple avantageusement utilisables comme « gommes de découplage » dans les zones du pneumatique (dites « zones de découplage ») ayant pour fonction d'assurer un découplage mécanique entre deux parties différentes dudit pneumatique, ces zones étant de manière connue exposée aux risques d'échauffement, et donc de réversion les plus importants. Elles peuvent également avantageusement constituer les profilés annulaires de gomme utilisés pour rigidifier les flancs de pneumatiques conçus pour un roulage à plat (« run-flat tyres » - voir à titre d'exemple US 5 427 166).

Par « élastomère isoprénique », on entend de manière connue un homopolymère ou un copolymère d'isoprène, en d'autres termes un élastomère diénique choisi dans le groupe constitué par le caoutchouc naturel (NR), les polyisoprènes de synthèse (IR), les différents copolymères d'isoprène et les mélanges de ces élastomères. Parmi les copolymères d'isoprène, on citera en particulier les copolymères d'isobutène-isoprène (caoutchouc butyle -IIR), d'isoprène-styrène (SIR), d'isoprène-butadiène (BIR) ou d'isoprène-butadiène-styrène (SBIR). Cet élastomère isoprénique est de préférence du caoutchouc naturel ou un polyisoprène cis-1,4 de synthèse ; parmi ces polyisoprènes de synthèse, sont utilisés de préférence des polyisoprènes ayant un taux (% molaire) de liaisons cis-1,4 supérieur à 90%, plus préférentiellement encore supérieur à 98%.

Selon un autre mode de réalisation préférentiel de l'invention, notamment lorsqu'elle est destinée à un flanc de pneumatique, à une gomme intérieure étanche de pneumatique sans chambre (ou autre élément imperméable à l'air), la composition conforme à l'invention peut contenir au moins un élastomère diénique essentiellement saturé, en particulier au moins un copolymère EPDM ou un caoutchouc butyle (éventuellement chloré ou bromé), que ces copolymères soient utilisés seuls ou en mélange avec des élastomères diéniques fortement insaturés tels que cités précédemment, notamment NR ou IR, BR ou SBR.

Les compositions de l'invention peuvent contenir un seul élastomère diénique ou un mélange de plusieurs élastomères diéniques, le ou les élastomères diéniques pouvant être utilisés en association avec tout type d'élastomère synthétique autre que diénique, voire avec des polymères autres que des élastomères, par exemple des polymères thermoplastiques.

### II-2. Charge renforçante

On peut utiliser tout type de charge renforçante connue pour ses capacités à renforcer une composition de caoutchouc utilisable pour la fabrication de pneumatiques, par exemple une charge organique tel que du noir de carbone, ou encore une charge inorganique renforçante telle que de la silice à laquelle sera associé dans ce deuxième cas un agent de couplage.

Comme noirs de carbone conviennent tous les noirs de carbone, notamment les noirs du type HAF, ISAF, SAF conventionnellement utilisés dans les pneumatiques (noirs dits de grade pneumatique). Parmi ces derniers, on citera plus particulièrement les noirs de carbone renforçants des séries 100, 200 ou 300 (grades ASTM), comme par exemple les noirs N115, N134, N234, N326, N330, N339, N347, N375, ou encore, selon les applications visées, les noirs de séries plus élevées (par exemple N660, N683, N772).

Par « charge inorganique renforçante », doit être entendu ici, de manière connue, toute charge inorganique ou minérale, quelles que soient sa couleur et son origine (naturelle ou de synthèse), encore appelée charge « blanche », charge « claire » ou encore charge « non-noire » (« non-black filler ») par opposition au noir de carbone, cette charge inorganique étant capable de renforcer à elle seule, sans autre moyen qu'un agent de couplage intermédiaire, une composition de caoutchouc destinée à la fabrication de pneumatiques, en d'autres termes apte à remplacer, dans sa fonction de renforcement, un noir de carbone conventionnel de grade pneumatique ; une telle charge se caractérise généralement, de manière connue, par la présence de groupes hydroxyle (-OH) à sa surface.

Comme charges inorganiques renforçantes conviennent notamment des charges minérales du type siliceuse, en particulier de la silice (SiO₂) ou du type alumineuse, en particulier de l'alumine (Al₂O₃). La silice utilisée peut être toute silice renforçante connue de l'homme du métier, notamment toute silice précipitée ou pyrogénée présentant une surface BET ainsi qu'une surface spécifique CTAB toutes deux inférieures à 450 m²/g, de préférence de 30 à 400 m²/g. A titres de silices précipitées hautement dispersibles (dites « HDS »), on citera par exemple les silices Ultrasil 7000 et Ultrasil 7005 de la société Degussa, les silices Zeosil 1165MP, 1135MP et 1115MP de la société Rhodia, la silice Hi-Sil EZ150G de la société PPG, les silices Zeopol 8715, 8745 et 8755 de la Société Huber. Comme exemples d'alumines renforçantes, on peut citer les alumines « Baikalox » « A125 » ou « CR125 » de la société Baïkowski, «APA-100RDX» de Condea, « Aluminoxid C » de Degussa) ou « AKP-G015» de Sumitomo Chemicals.

Pour coupler la charge inorganique renforçante à l'élastomère diénique, on utilisera de manière bien connue un agent de couplage (ou agent de liaison) au moins bifonctionnel destiné à assurer une connexion suffisante, de nature chimique et/ou physique, entre la charge inorganique (surface de ses particules) et l'élastomère diénique, en particulier des organosilanes ou des polyorganosiloxanes bifonctionnels.

De manière préférentielle, le taux de charge renforçante totale (noir de carbone et/ou charge inorganique renforçante) est compris entre 20 et 200 pce, plus préférentiellement entre 30 et 150 pce (parties en poids pour cent parties d'élastomère), l'optimum étant différent selon les applications visées : le niveau de renforcement attendu sur un pneumatique vélo, par exemple, est de manière connue nettement inférieur à celui exigé sur un pneumatique apte à rouler à grande vitesse de manière soutenue, par exemple un pneu moto, un pneu pour véhicule de tourisme ou pour véhicule utilitaire tel que Poids lourd.

### II-3. Itaconimidomaléimide

Les compostions de caoutchouc de l'invention ont pour caractéristique nouvelle et inventive d'utiliser, à titre d'agent agent antiréversion, un composé itaconimidomaléimide comportant à la fois une fonction maléimide et une fonction itaconimide, répondant à la formule (I) suivante :

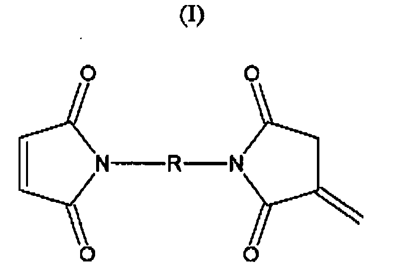

Le radical R est un radical hydrocarboné quelconque, aromatique ou aliphatique, cyclique ou acyclique, substitué ou non substitué, linéaire ou ramifié, qui comporte de 1 à 25 atomes de carbone, et éventuellement un ou plusieurs hétéroatome(s) choisi(s) parmi O, N et S.

Plus préférentiellement, R est choisi dans le groupe constitué par les alkylènes ayant de 1 à 20 atomes de carbone, les cycloalkylènes ayant de 6 à 24 atomes de carbone, les arylènes ayant de 6 à 18 atomes de carbone et les aralkylènes ayant de 7 à 25 atomes de carbone.

A titre d'exemples d'itaconimidomaléimides répondant à cette définition, on citera notamment ceux appartenant au groupe préférentiel constitué par le N-(itaconimido-éthyl) maléimide, le N-(itaconimido-hexaméthyl) maléimide, le N-(itaconimido-dodécaméthyl) maléimide, le N-(itaconimido-oxy-dipropyl) maléimide, le N-(itaconimido-1,3-cyclohexyl) maléimide, le N-(itaconimido-1,4-cyclohexyl) maléimide, le N-(1'-itaconimido-3,3'-diméthyl-4,4'-biphényl) maléimide, le N-(itaconimido-m-phényl) maléimide, le N-(itaconimido-p-phényl) maléimide, le N-(itaconimido-o-phényl) maléimide, le N-(itaconimido-1,3-naphtyl) maléimide, le N-(itaconimido-1,4-naphtyl) maléimide, le N-(itaconimido-1,5-naphtyl) maléimide, le N-(3-itaconimido-4,6-diméthyl-phényl) maléimide, le N-(3-itaconimido-4-méthyl-phényl) maléimide, le N-(3-itaconimido-6-méthyl-phényl) maléimide; le N-(3-itaconimido-2-méthyl-phényl) maléimide; le N-(1'-itaconimido-4,4'-méthylène-bi-phényl) maléimide, le N-[2-(méthylène-itaconimido)-phényl]-méthylène-maléimide; le N-[3-(méthylène-itaconimido)-phényl]-méthylène-maléimide ; le N-[4-(méthylène-itaconimido)-phényl]-méthylène-maléimide ; le N-(itaconimido-oxy-dipropyl) maléimide, le N-(itaconimido-oxy-di-p-phényl) maléimide, le N-(1'-itaconimido-4,4'-dithiobiphényl) maléimide, et les mélanges de ces composés.

Selon un mode de réalisation de l'invention particulièrement préféré, l'itaconimidomaléimide est plus préférentiellement choisi dans le groupe constitué par le N-(itaconimido-m-phényl) maléimide, le N-(itaconimido-p-phényl) maléimide, le N-(itaconimido-o-phényl) maléimide, le N-(3-itaconimido-4,6-diméthyl-phényl) maléimide, le N-(3-itaconimido-4-méthyl-phényl) maléimide, le N-(3-itaconimido-6-méthyl-phényl) maléimide, le N-(3-itaconimido-2-méthylphényl) maléimide, le N-(1'-itaconimido-4,4'-méthylène-bi-phényl) maléimide, le N-[2-(méthylène-itaconimido)-phényl]-méthylène-maléimide, le N-[3-(méthylène-itaconimido)-phényl]-méthylène-maléimide, le N-[4-(méthylène- itaconimido)-phényl]-méthylène-maléimide, et les mélanges de ces composés.

Selon un mode de réalisation plus particulièrement préféré, R est un groupe phénylène, l'itaconimidomaléimide sélectionné étant encore plus préférentiellement le N-(itaconimido-p-phényl) maléimide.

L'itaconimidomaléimide est présent dans la composition selon l'invention à un taux préférentiel compris entre 0,1 et 10 pce. En dessous du minimum indiqué, l'effet technique visé peut être insuffisant alors qu'au-delà du maximum indiqué, on s'expose à un double risque pour les compositions de plastification à l'état cru et d'une rigidification excessive à l'état cuit. Pour toutes ces raisons, on utilise un taux plus préférentiellement compris dans un domaine de 0,2 à 5 pce. Une quantité comprise dans un domaine de 0,2 à 2,5 pce s'est révélée particulièrement bien adaptée à l'application pneumatique.

Les composés de formule (I) décrits ci-dessus peuvent être préparés par double réaction d'addition-élimination d'une diamine en présence d'anhydride maléique et d'anhydride itaconique, suivie d'une étape de cyclisation.

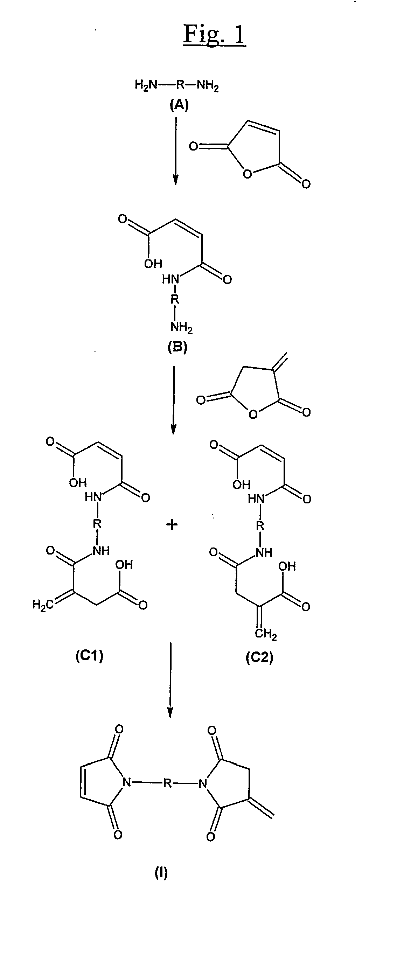

A titre d'exemple, la figure 1 annexée illustre une voie de synthèse préférée, comportant les étapes suivantes s'inspirant de méthodes connues (R ayant les significations précédentes) :
- a) on part d'une diamine (ci-après produit A) de formule (A) :

   H₂N-R-NH₂
- b) on la met en présence d'anhydride maléique dans un solvant organique inerte, une première réaction d'addition-élimination se produit pour conduire à un acide maléamique de formule (B), non isolé, qui, mis en présence d'anhydride itaconique dans un solvant organique inerte, va engendrer une seconde réaction d'addition-élimination similaire à la précédente et conduire à un diacide maléamique et itaconamique (produit C), pouvant se présenter sous la forme de deux régioisomères (produits C1 et C2) de formules respectives (C1) et (C2) : 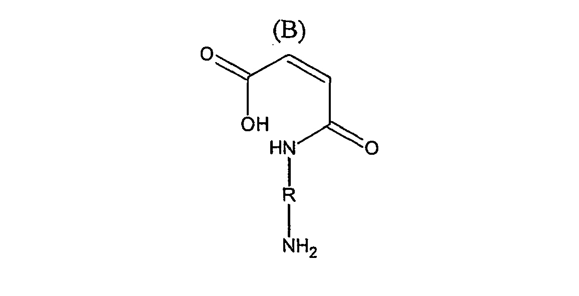 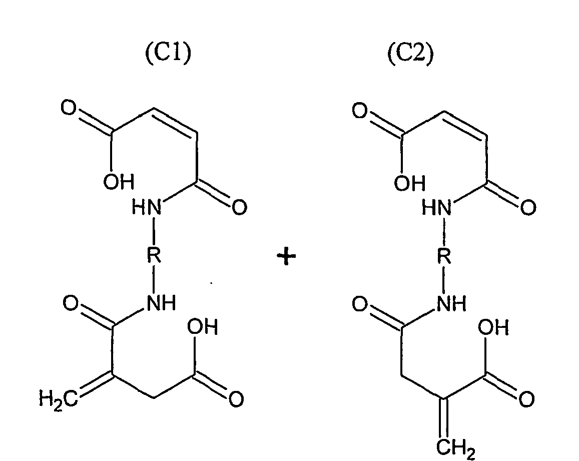
- c) puis on réalise une étape de cyclisation du produit C, en présence d'un solvant organique anhydre (par exemple du toluène), d'un catalyseur du type acide de Lewis (par exemple ZnCl₂) et d'un agent de cyclisation tel que l'hexaméthyldisilazane (HMDS) pour générer le produit de formule (I) visé : 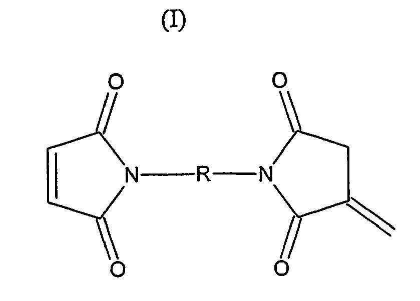 L'homme du métier comprendra aisément qu'une variante possible de ce schéma de synthèse serait de faire réagir la diamine dans un premier temps avec l'anhydride itaconique, puis dans un deuxième temps avec l'anhydride maléique, en d'autres termes d'inverser l'ordre des réactifs dans l'étape b) ci-dessus.

### II-4. Système de vulcanisation

Le système de vulcanisation proprement dit est à base de soufre (ou d'un agent donneur de soufre) et d'un accélérateur primaire de vulcanisation. A ce système de vulcanisation de base viennent s'ajouter, incorporés au cours de la première phase non-productive et/ou au cours de la phase productive telles que décrites ultérieurement, divers accélérateurs secondaires ou activateurs de vulcanisation connus tels qu'oxyde de zinc, acide stéarique ou composés équivalents, dérivés guanidiques (en particulier diphénylguanidine).

Le soufre est utilisé à un taux préférentiel compris entre 0,5 et 10 pce, plus préférentiellement compris entre 1 et 8 pce, en particulier entre 1 et 6 pce lorsque la composition de l'invention est destinée, selon un mode préférentiel de l'invention, à constituer une gomme interne de pneumatique, notamment une gomme de découplage.

L'accélérateur primaire de vulcanisation est quant à lui utilisé à un taux préférentiel compris entre 0,5 et 10 pce, plus préférentiellement compris entre 0,5 et 5,0 pce.

Un tel accélérateur, on le sait, doit permettre une réticulation des compositions de caoutchouc dans des temps industriellement acceptables, tout en préservant un délai minimum de sécurité ("temps de grillage") au cours duquel les compositions peuvent être mises en forme sans risque de vulcanisation prématurée ("grillage").

On peut utiliser tout composé susceptible d'agir comme accélérateur de vulcanisation des élastomères diéniques en présence de soufre.

Conviennent en particulier les accélérateurs du type thiazoles ainsi que leurs dérivés de formule (II): 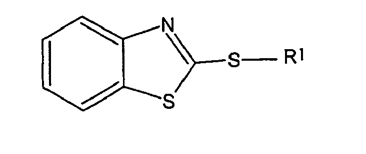 dans laquelle R¹ représente un atome d'hydrogène, un groupement 2-mercaptobenzothiazyle de formule (III): 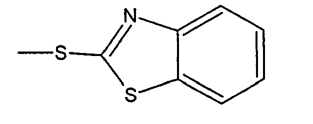 ou encore un groupement de formule (IV) : 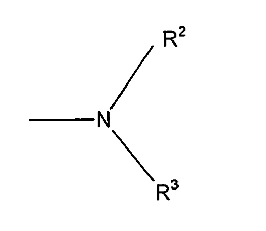 dans laquelle R² et R³ représentent indépendamment un atome d'hydrogène, un groupement 2-mercaptobenzothiazyle (formule III), un groupement alkyle en C₁-C₄ ou un groupement cycloalkyle en C₅-C₈, comportant de préférence 6 maillons, ledit cycle pouvant comporter au moins un hétéroatome tel que S, O ou N.

Des accélérateurs thiazoles et dérivés préférentiels sont notamment choisis dans le groupe constitué par le 2-mercaptobenzothiazole, le disulfure de 2-mercapto-benzothiazyle, le N-cyclohexyl-2-benzothiazyle sulfénamide, N,N-dicyclohexyl-2-benzothiazyle sulfénamide, N-ter-butyl-2-benzothiazyle sulfénamide, N-cyclohexyl-2-benzothiazyle sulfénimide, N-terbutyl-2-benzothiazyle sulfénimide et les mélanges de ces composés.

Comme accélérateurs conviennent également les composés de la famille des thiurames (formule V) et les dérivés dithiocarbamates de zinc (formule VI) : 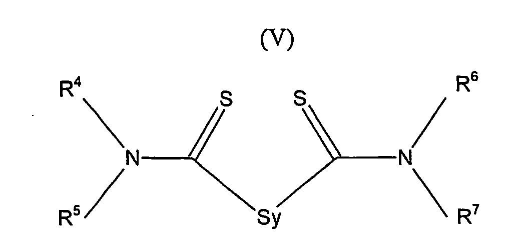 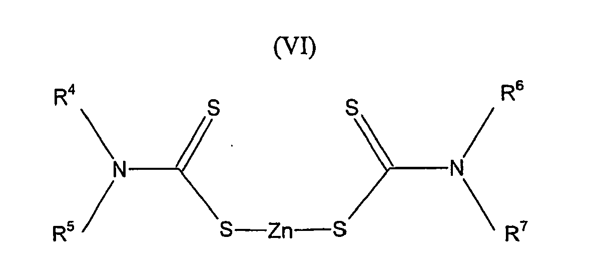 dans lesquelles y varie de 1 à 4, y est plus particulièrement égal à 1 ou 2 ; R⁴, R⁵, R⁶ et R⁷ représentent chacun indépendamment un groupement alkyle comprenant de 1 à 8 atomes de carbone, un groupement benzyle, une combinaison entre R⁴ et R⁵ et une combinaison entre R⁶ et R⁷ pour former un groupement cyclique pentaméthylène ou un groupement cyclique méthyl-pentaméthylène et dans lesquelles R⁴ et R⁵ et R⁶ et R⁷ sont reliés entre eux.

Des accélérateurs de types thiurames sont notamment choisis dans le groupe préférentiel constitué par le monosulfure de tétraméthyl-thiurame, le disulfure de tétraméthyl-thiurame, le disulfure de tétraéthyl-thiurame, le disulfure de tétrabutyl-thiurame, le disulfure de tétra-iso-butyl-thiurame, le disulfure de tétrabenzyl-thiurame et les mélanges de ces composés. Parmi eux, le disulfure de tétrabenzyl-thiurame est retenu de manière plus préférentielle.

A titre d'autres exemples d'accélérateurs utilisables dans les compositions de l'invention, on citera les dithiocarbamates de zinc, en particulier le tétraméthyl dithiocarbamate de zinc, le tétraéthyl dithiocarbamate de zinc et que le tétrabenzyle dithiocarbamate de zinc. Parmi eux, le tétrabenzyl dithiocarbamate de zinc est plus préférentiellement retenu.

Pour résumer, les accélérateurs primaires de vulcanisation utilisés dans la composition selon l'invention sont plus préférentiellement choisis dans le groupe constitué par disulfure de 2-mercaptobenzothiazyle (en abrégé "MBTS"), N-cyclohexyl-2-benzothiazyle sulfénamide (en abrégé "CBS"), N,N-dicyclohexyl-2-benzothiazyle sulfénamide (en abrégé "DCBS"), N-terbutyl-2-benzothiazyle sulfénamide (en abrégé "TBBS"), N-ter-butyl-2-benzothiazyle sulfénimide (en abrégé "TBSI") et les mélanges de ces composés.

### II-5. Additifs divers

Bien entendu, les compositions élastomériques conformes à l'invention peuvent comporter également tout ou partie des additifs usuels utilisés dans des compositions de caoutchouc destinées à la fabrication de pneumatique, comme par exemple des plastifiants ou des huiles d'extension, que ces derniers soient de nature aromatique ou non-aromatique, des pigments, des agents de protection tels que cires anti-ozone, anti-ozonants chimiques, anti-oxydants, agents anti-fatigue, des promoteurs d'adhésion, des activateurs de couplage, des résines renforçantes, des accepteurs et/ou donneurs de méthylène, voire d'autres agents antiréversion, par exemple des bismaléimides conventionnels.

De préférence, ces compositions comportent, à titre d'agent plastifiant préférentiel non aromatique ou très faiblement aromatique, au moins un composé choisi dans le groupe constitué par les huiles naphténiques, paraffiniques, huiles MES, huiles TDAE, les esters (en particulier trioléates) de glycérol, les résines plastifiantes hydrocarbonées présentant de préférence une valeur élevée de Tg (de préférence supérieure à 30°C), et les mélanges de tels composés.

Dans le cas où la charge renforçante utilisée est une charge inorganique, on pourra utiliser avantageusement des agents de recouvrement d'une telle charge inorganique, plus généralement des agents d'aide à la mise en oeuvre susceptibles de manière connue, grâce à une amélioration de la dispersion de la charge inorganique dans la matrice de caoutchouc et à un abaissement de la viscosité des compositions, d'améliorer leur faculté de mise en oeuvre à l'état cru.

### II-6. Préparation des compositions de caoutchouc

Les compositions sont fabriquées dans des mélangeurs appropriés, en utilisant deux phases de préparation successives bien connues de l'homme du métier : une première phase de travail ou malaxage thermomécanique (phase dite "non-productive") à haute température, jusqu'à une température maximale comprise entre 110°C et 190°C, de préférence entre 130°C et 180°C, suivie d'une seconde phase de travail mécanique (phase dite "productive") jusqu'à une plus basse température, typiquement inférieure à 110°C, par exemple entre 40°C et 100°C, phase de finition au cours de laquelle est incorporé le système de vulcanisation.

Le procédé conforme à l'invention pour préparer une composition de caoutchouc présentant une résistance à la réversion améliorée comporte les étapes suivantes :
- incorporer à un élastomère diénique, au cours d'une première étape dite "non-productive", au moins une charge renforçante, en malaxant thermomécaniquement le tout, en une ou plusieurs fois, jusqu'à atteindre une température maximale comprise entre 110°C et 190°C;
- refroidir l'ensemble à une température inférieure à 100°C ;
- incorporer ensuite, au cours d'une seconde étape dite "productive", le système de vulcanisation ;
- malaxer le tout jusqu'à une température maximale inférieure à 110°C,
et il est caractérisé en ce qu'est en outre incorporé, au cours de l'une quelconque des étapes du procédé, un composé itaconimidomaléimide de formule (I) précitée.

A titre d'exemple, la phase non-productive est conduite en une seule étape thermomécanique au cours de laquelle on introduit, dans un mélangeur approprié tel qu'un mélangeur interne usuel, dans un premier temps tous les constituants de base nécessaires (élastomère diénique, charge renforçante et agent de couplage si nécessaire, éventuellement tout ou partie du composé itaconimidomaléimide), puis dans un deuxième temps, par exemple après une à deux minutes de malaxage, les autres additifs, éventuels agents de recouvrement ou de mise en oeuvre complémentaires, à l'exception du système de vulcanisation. La durée totale du malaxage, dans cette phase non-productive, est de préférence comprise entre 1 et 15 min.

Après refroidissement du mélange ainsi obtenu, on incorpore alors dans un mélangeur externe tel qu'un mélangeur à cylindres, maintenu à basse température (par exemple entre 40°C et 100°C), le système de vulcanisation ainsi que le composé itaconimidomaléimide (tout ou la partie restante, le cas échéant). L'ensemble est alors mélangé (phase productive) pendant quelques minutes, par exemple entre 2 et 15 minutes.

La composition finale ainsi obtenue peut ensuite être calandrée, par exemple sous la forme d'une feuille, d'une plaque ou encore extrudée, par exemple pour former un profilé de caoutchouc utilisé pour la fabrication d'un produit semi-fini pour pneumatique, tel que nappes, bandes, sous-couches, divers blocs de caoutchouc, renforcés ou non de renforts textiles ou métalliques, destinés à former une partie de la structure du pneumatique.

La vulcanisation (ou cuisson) peut ensuite être conduite de manière connue à une température généralement comprise entre 130°C et 200°C, de préférence sous pression, pendant un temps suffisant qui peut varier par exemple entre 5 et 90 min en fonction notamment de la température de cuisson, du système de vulcanisation adopté et de la cinétique de vulcanisation de la composition considérée.

L'invention concerne les compositions de caoutchouc précédemment décrites tant à l'état dit "cru" (i.e. avant cuisson) qu'à l'état dit "cuit" ou vulcanisé (i.e. après vulcanisation).

### III. EXEMPLES DE REALISATION DE L'INVENTION

### III-1. Synthèse de l'itaconimidomaléimide

On prépare le N-(p-itaconimidophényl)-maléimide par réaction de la 1,4-phénylènediamine sur de l'anhydride maléique puis de l'anhydride itaconique ; le diacide alors généré est ensuite cyclisé en s'inspirant d'un procédé de synthèse de dérivés N-alkyl- et N-arylimides tel que décrit dans J. Org. Chem., Vol. 62 No. 8, 2652-2654, 1997.

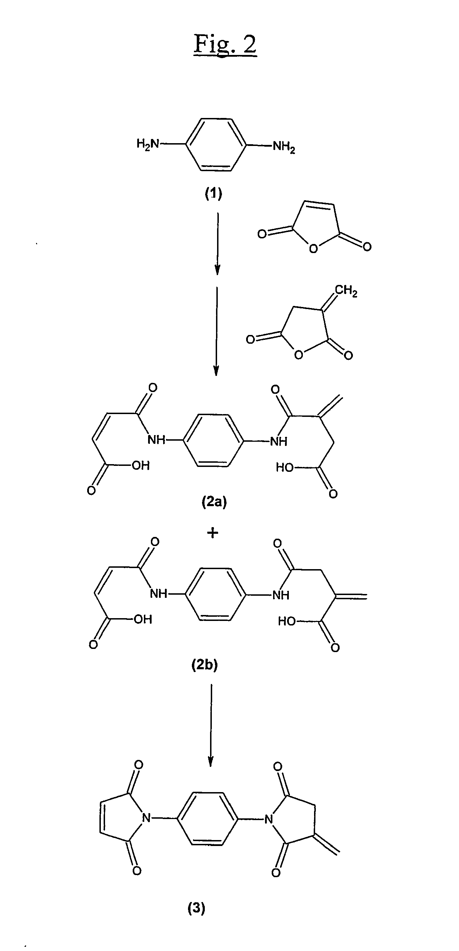

Plus précisément, cette synthèse est opérée en deux étapes telles que schématisées sur la Figure 2 annexée.

### A) 1^{ère} étape : synthèse du diacide 1,4-phénylène-N-itaconamique-N-maléamique (produits (2a) et (2b) sur figure 2)

Dans un bicol de 500 mL, on ajoute la 1,4-phénylènediamine **(1)** (10.50 g soit 0,094mol - 1 équivalent) et 222mL de THF (tétrahydrofurane). A cette solution est ajoutée en 10 minutes, à température ambiante, une solution d'anhydride maléique (9.34g soit 0,094mol- 1 éq) dans 48mL de THF, sous atmosphère d'argon. Au bout d'une 1h 30 d'agitation à la température ambiante, on additionne au milieu, devenu hétérogène et de couleur orange, une solution d'anhydride itaconique (16.68mL, soit 0,141mol - 1.5éq) dans 60mL de THF en 10minutes. Le milieu est abandonné sous agitation à la température ambiante pendant 16heures. Le précipité est filtré sur un verre fritté puis rincé à l'éther éthylique. Celui-ci est séché à la température ambiante; on obtient ainsi 30g de diacide 1,4-phénylène-N-itaconamique-N'-maléamique sous la forme d'une fine poudre jaune claire.

### B) 2^{ème} étape : synthèse du N-(p-itaconimidophényl)maléimide (produit (3) sur figure 2)

Dans un tricol sec et sous atmosphère inerte, on introduit le diacide **(2a** et **2b)** (11.7 g soit 0,0347mol - 1 éq) et 1.5 L de toluène anhydre. On soumet le mélange formé à une agitation mécanique pendant quelques minutes à la température ambiante, puis on ajoute en une seule fois du ZnCl₂ (10.16 g soit 0, 728 mol - 2,1 éq). On porte le mélange réactionnel à 80°C puis on additionne en 10minutes l'HMDS (18.16 mL soit 0,0869 mol - 2.5 éq). On laisse sous agitation vive et chauffage pendant 24 heures, puis on évapore à sec à l'aide d'un évaporateur rotatif (bain d'eau à 80°C sous 1 mm Hg). Le produit brut obtenu est repris dans 745 mL de dichlorométhane à 50°C pendant 20 min pour dissoudre le maximum de produit, on filtre à chaud sur un verre fritté, le précipité beige recueilli correspondant au ZnCl₂. La phase organique isolée est alors lavée avec respectivement 100mL d'une solution saturée de NaHCO₃ une solution saturée de Na2CO3, séchée sur MgSO₄, filtrée puis évaporée.

On obtient ainsi 1.70g de N-(p-itaconimidophényl)maléimide **(3)** sous la forme d'une poudre très pulvérulente beige.

### III-2. Préparation des compositions

On procède pour les essais qui suivent de la manière suivante: on introduit dans un mélangeur interne à palettes usuelles du type "Banburry" (capacité : environ 3,5 litres), rempli à 70% et dont la température initiale de cuve est d'environ 60°C, l'élastomère diénique (ou le mélange d'élastomères diéniques, le cas échéant), la charge renforçante (noir de carbone) puis, après une à deux minutes de malaxage, les divers autres ingrédients à l'exception du système de vulcanisation et de l'agent antiréversion.

On conduit alors un travail thermomécanique (phase non-productive) en une étape (durée totale du malaxage égale à environ 5 min), jusqu'à atteindre une température maximale de "tombée" d'environ 160°C. On récupère le mélange ainsi obtenu, on le refroidit puis on ajoute le système de vulcanisation et (s'il est présent dans la composition) l'agent antiréversion sur un mélangeur externe (homo-finisseur) à 40°C, en mélangeant le tout (phase productive) pendant 3 à 4 minutes sur cet outil à cylindres.

Les compositions ainsi obtenues sont ensuite calandrées sous la forme de plaques (épaisseur de 2 à 3 mm) ou de feuilles fines de caoutchouc pour la mesure de leurs propriétés physiques ou mécaniques, ou extrudées pour former des profilés utilisables directement, après découpage et/ou assemblage aux dimensions souhaitées, comme produit semi-fini pour pneumatique.

### III-3. Essais de caractérisation - Résultats

Cet essai a pour but de démontrer la résistance améliorée à la réversion d'une composition selon l'invention destinée à constituer une gomme de découplage située entre l'armature de sommet et l'armature de carcasse radiale d'un pneumatique du type génie civil.

Cette composition selon l'invention est comparée à deux compositions témoins, comportant ou non un agent antiréversion, les trois compositions testées étant identiques aux différences près qui suivent :
- composition T-1 : témoin sans agent antiréversion ;
- composition T-2 : témoin avec agent antiréversion conventionnel (bis-maléimide) ;
- composition C-1 : composition selon l'invention (itaconimidomaléimide).

Le composé bismaléimide utilisé dans la composition témoin T-2 est le méta-phénylènebismaléimide (en abrégé "MPBM"), bien connu de l'homme du métier et répondant à la formule particulière suivante :

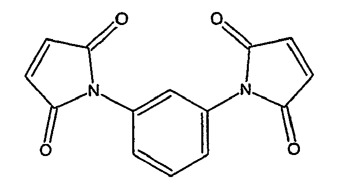

On rappelle que l'itaconimidomaléimide utilisé dans la composition selon l'invention C-1 répond à la formule particulière qui suit :

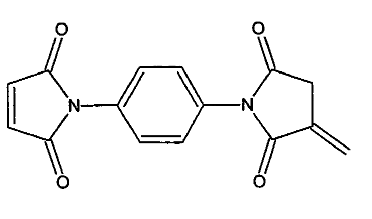

Ainsi, la caractéristique essentielle distinguant les deux composés ci-dessus, et donc la composition selon l'invention C-1 de la composition témoin T-2, est la présence sur un et un seul des deux groupes cycliques imide de la double liaison en position exo.

Les deux composés maléimide sont utilisés à un taux isomolaire.

Les tableaux 1 et 2 donnent la formulation des différentes compositions (tableau 1 - taux des différents produits exprimés en pce), les propriétés après cuisson, les propriétés rhéométriques ainsi que différents paramètres mesurant la réversion. Pour apprécier la résistance à la réversion des compositions, on suit l'évolution du couple rhéométrique après 60 min à 150°C. La stabilité thermique des compositions est également appréciée par l'évolution du module sécant nominal à 100% et à 300% d'allongement, entre la mesure à l'optimum de cuisson et après une cuisson prolongée de 6 heures (température de 150°C).

A la lecture du tableau 2, on note tout d'abord que les propriétés usuelles des compositions, après cuisson, sont sensiblement identiques : modules en traction (MA100, MA300) et rapport MA300/MA100 (indice connu de renforcement) très proches, propriétés rhéométriques assez voisines.

Mais ce sont les paramètres de mesure de la réversion qui démontrent tout l'intérêt de la composition selon l'invention par rapport aux deux compositions témoin.

La stabilité thermique de la composition C-1 apparaît remarquable, nettement supérieure à celle observée sur les deux témoins T-1 et T-2, ceci quel que soit le paramètre utilisé (conformément aux indications données dans le paragraphe 1-5) : la perte ΔR₆₀ comme les évolutions de modules ΔMA100 et ΔMA300 indiquent toutes très clairement que le phénomène de réversion est inexistant sur la composition de l'invention, alors qu'il reste relativement prononcé sur la composition témoin T-2 malgré la présence de bismaléimide conventionnel (MPBM).

Ainsi la présence de la double liaison en exo du cycle itaconimide sur un des groupes imide de la molécule confère aux compositions de caoutchouc de l'invention cette stabilité thermique inattendue.

**Tableau 1**

| Composition N°: | T-1 | T-2 | C-1 |
|---|---|---|---|
| NR (1) | 100 | 100 | 100 |
| Noir de carbone (2) | 35 | 35 | 35 |
| ZnO (3) | 5 | 5 | 5 |
| Acide Stéarique (4) | 1.5 | 1.5 | 1.5 |
| anti-oxydant (5) | 1.5 | 1.5 | 1.5 |
| bis maléimide (6) | - | 1.5 | - |
| itaconimido-maléimide (7) | - | - | 1.6 |
| soufre (8) | 1.6 | 1.6 | 1.6 |
| accélérateur (9) | 0.6 | 0.6 | 0.6 |

| | | | |
|---|---|---|---|
| (1) caoutchouc naturel (peptisé) ; (2) N375 (société Cabot) ; (3) oxyde de zinc (grade industriel - société Umicore) ; (4) stéarine ("Pristerene 4931 ") de la société Uniqema ; (5) N-1,3-diméthylbutyl-N-phényl-para-phénylènediamine ("Santoflex 13" de la société Flexsys) ; (6) MPBM (société Safic Alcan) ; (7) N-(p-maléimidophényl)-itaconimide synthétisé selon paragraphe III-1 ; (8) soufre (soufre synthétique de la société Solvay) ; (9) TBBS (N-tertiobutyl-2-benzothiazyle-sulfénamide) - ("Santocure TBBS" de la société Flexsys). | | | |

**Tableau 2**

| Composition | T1 | T2 | C1 |
|---|---|---|---|
| *Propriétés rhéométriques à 150°C:* | | | |
| Cmin (dN.m) | 0.5 | 0.5 | 0.5 |
| Cmax (dN.m) | 6.1 | 9.3 | 8.0 |
| ΔCouple (dN.m) | 5.6 | 8.7 | 7.4 |

| *propriétés après cuisson à l'optimum à 150°C:* | | | |
|---|---|---|---|
| MA100 (MPa) | 1.48 | 1.71 | 1.53 |
| MA300 (MPa) | 1.84 | 2.27 | 1.94 |
| MA300/MA100 | 1.24 | 1.32 | 1.26 |

| *Réversion (1 heure à 150°C)* | | | |
|---|---|---|---|
| ΔR60(%) | -14 | -10 | -5 |
| □ | | | |

| *Propriétés après cuisson prolongée de 6 heures à 150°C et réversion:* | | | |
|---|---|---|---|
| MA100 (MPa) | 1.12 | 1.42 | 1.58 |
| MA300 (MPa) | 1.25 | 1.70 | 1.96 |
| ΔMA100(%) | -24 | -17 | 3 |
| ΔMA300(%) | -32 | -25 | 1 |

## Revendications

1. Composition de caoutchouc utilisable pour la fabrication de pneumatiques, à base d'au moins (i) un élastomère diénique, (ii) une charge renforçante, (iii) un système de vulcanisation et (i.v) un composé maléimide, **caractérisé en ce que** ledit composé maléimide est un itaconimidomaléimide de formule (R radical hydrocarboné comportant de 1 à 25 atomes de carbone et éventuellement un ou plusieurs hétéroatome(s) choisi(s) parmi O, N et S) :

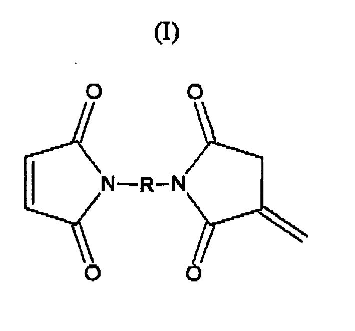

2. Composition selon la revendication 1, R étant choisi dans le groupe constitué par les alkylènes ayant de 1 à 20 atomes de carbone, les cycloalkylènes ayant de 6 à 24 atomes de carbone, les arylènes ayant de 6 à 18 atomes de carbone et les aralkylènes ayant de 7 à 25 atomes de carbone.

3. Composition selon la revendication 2, l'itaconimidomaléimide étant choisi dans le groupe constitué par le N-(itaconimido-m-phényl) maléimide, le N-(itaconimido-p-phényl) maléimide, le N-(itaconimido-o-phényl) maléimide, le N-(3-itaconimido-4,6-diméthylphényl) maléimide, le N-(3-itaconimido-4-méthyl-phënyl) maléimide, le N-(3-itaconimido-6-méthyl-phényl) maléimide, le N-(3-itaconimido-2-méthyl-phényl) maléimide, le N-(1'-itaconimido-4,4'-méthylène-bi-phényl) maléimide, le N-[2-(méthylène-itaconimido)-phényl]-méthylène-maléimide, le N-[3-(méthyléne-itaconimido)-phényl]-méthyléne-maléimide, le N-[4-(méthylène-itaconimido)-phényl]-méthylène-maléimide, et les mélanges de ces composés.

4. Composition selon les revendications 2 ou 3, R étant un groupe phénylène.

5. Composition selon la revendication 4, l'itaconimidomaléimide étant le N-(p-itaconimidophényl)-maléinide.

6. Composition selon l'une quelconque des revendications 1 à5, l'élastomère diénique étant choisi dans le groupe constitué par les polybutadiènes, le caoutchouc naturel, les polyisoprènes de synthèse, les copolymères de butadiène, les copolymères d'isoprène et les mélanges de ces élastomères.

7. Composition selon l'une quelconque des revendications 1 à 6, la charge renforçante étant présente à un taux compris entre 20 et 200 pce, de préférence entre 30 et 150 pce (parties en poids pour cent d'élastomère diénique).

8. Composition selon l'une quelconque des revendications 1 à 7, la charge renforçante étant majoritairement une charge inorganique, de préférence de la silice.

9. Composition selon l'une quelconque des revendication 1 à 7, la charge renforçante étant majoritairement une charge organique, de préférence du noir de carbone.

10. Composition selon l'une quelconque des revendications 6 à 9, l'élastomère diénique étant un élastomère isoprénique, de préférence un polyisoprène de synthèse ou du caoutchouc naturel.

11. Composition selon l'une quelconque des revendications 1 à 10, le taux d'itaconimidomaléimide étant compris entre 0,1 et 10 pce.

12. Composition selon la revendication 11, le taux d'itaconimidomaléimide étant compris dans un domaine de 0,2 à 2,5 pce.

13. Composition selon l'une quelconque des revendications 1 à 12, **caractérisée en ce qu'**elle comporte en outre un accélérateur primaire de vulcanisation, choisi de préférence dans le groupe constitué par disulfure de 2-mercaptobezuothiazyle, N-cyclohexyl-2-benzothiazyle sulfénamide, N,N-dicyclohexyl-2-benzothiazyle sulfénamide, N-ter-butyl-2-benzothiazyle sulfénamide, N-ter-butyl-2-benzothiazyle sulfénimide et les mélanges de ces composés.

14. Procédé pour préparer une composition de caoutchouc utilisable pour la fabrication de pneumatiques et présentant une résistance à la réversion améliorée, cette composition étant à base d'un élastomère diénique, d'une charge renforçante et d'un système de vulcanisation, ledit procédé comportant les étapes suivantes :
• incorporer à un élastomère diénique, au cours d'une première étape dite "non-productive", au moins une charge renforçante, en malaxant thermomécaniquement le tout, en une ou plusieurs fois, jusqu'à atteindre une température maximale comprise entre 110°C et 190°C ;
• refroidir l'ensemble à une température inférieure à 100°C ;
• incorporer ensuite, au cours d'une seconde étape dite "productive", le système de vulcanisation ;
• malaxer le tout jusqu'à une température maximale inférieure à 110°C,
**caractérisé en ce qu'**est en outre incorporé, au cours de l'une quelconque des étapes du procédé, un composé itaconimidomaléimide de formule (I) (R radical hydrocarboné comportant de 1 à 25 atomes de carbone et éventuellement un ou plusieurs hétéroatome(s) choisi(s) parmi O, N et S) :

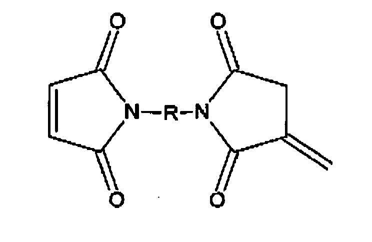

15. Procédé selon la revendication 14, R étant choisi dans le groupe constitué par les alkylènes ayant de 1 à 20 atomes de carbone, les cycloalkylènes ayant de 6 à 24 atomes de carbone, les arylènes ayant de 6 à 18 atomes de carbone et les aralkylènes ayant de 7 à 25 atomes'de carbone.

16. Procédé selon la revendication 15, l'itaconimidomaléimide étant choisi dans le groupe constitué par le N-(itaconimido-m-phényl) maléimide, le N-(itaconimido-p-phényl) maléimide, le N-(itaconimido-o-phényl) maléimide, le N-(3-itaconimido-4,6-diméthylphényl) maléimide, le N-(3-itaconimido-4-méthyl-phényl) maléimide, le N-(3-itaconimido-6-méthyl-phényl) maléimide, le N-(3-itaconimido-2-méthyl-pbényl) maléimide, le N-(1'-itaconimido-4,4'-méthylène-bi-phényl) maléimide, le N-[2-(méthylène-itaconimido)-phényl]-méthylène-maléimide, le N-[3-(méthylène-itaconimido)-phényl]-méthylène-maléimide, le N-[4-(méthylène-itaconimido)-phényl]-méthylène-maléimide, et les mélanges de ces composés.

17. Procédé selon les revendications 15 ou 16, R étant un groupe phénylène.

18. Procédé selon la revendication 17, l'itaconimidomaléimide étant le N-(p-itaconimidophényl) maléimide.

19. Procédé selon l'une quelconque des revendications 14 à 18, l'élastomère diénique étant choisi dans le groupe constitué par les polybutadiènes, le caoutchouc naturel, les polyisoprènes de synthèse, les copolymères de butadiène, les copolymères d'isoprène et les mélanges de ces élastomères.

20. Procédé selon l'une quelconque des revendications 14 à 19, la charge renforçante étant présente à un taux compris entre 20 et 200 pce, de préférence entre 30 et 150 pce (parties en poids pour cent d'élastomère diénique).

21. Procédé salon l'une quelconque des revendications 14 à 20, la charge renforçante étant majoritairement une charge inorganique, de préférence de la silice.

22. Procédé selon l'une quelconque des revendications 14 à 20, la charge renforçante étant majoritairement une charge organique, de préférence du noir de carbone.

23. Procédé selon l'une quelconque des revendications 19 à 22, l'élastomère diénique étant un élastomère isoprénique, de préférence un polyisoprène de synthèse ou du caoutchouc naturel.

24. Procédé selon l'une quelconque des revendications 14 à 23, le taux d'itaconimidomaléimide étant compris entre 0,1 et 10 pce.

25. Procédé selon la revendication 24, le taux de itaconimidomaléimide étant compris dans un domaine de 0,2 à 2,5 pce.

26. Procédé selon l'une quelconque des revendications 14 à 25, le système de vulcanisation comportant en outre un accélérateur primaire de vulcanisation, de préférence choisi dans le groupe constitué par disulfure de 2-mercaptobenzothiazyle, N-cyclohexyl-2-benzothiazyle sulfénamide, N,N-dicyclohexyl-2-benzothiazyle sulfénamide, N-ter-butyl-2-benzothiazyle sulfénamide, N-ter-butyl-2-benzothiazyle sulfénimide et les mélanges de ces composés.

27. Utilisation d'une composition selon l'une quelconque des revendications 1 à 13, pour la fabrication d'un article fini ou d'un produit semi-fini destiné à un système de liaison au sol de véhicule automobile.

28. Article fini ou produit semi-fini destiné à un système de liaison au sol de véhicule automobile, comportant une composition selon l'une quelconque des revendications 1 à 13.

## Claims

1. Rubber composition which can be used for the manufacture of tyres based on at least (i) one diene elastomer, (ii) one reinforcing filler, (iii) one vulcanization system and (iv) one maleimide compound, **characterized in that** the said maleimide compound is an itaconimidomaleimide of formula (R hydrocarbon radical comprising from 1 to 25 carbon atoms and optionally one or more heteroatom(s) chosen from O, N and S) :

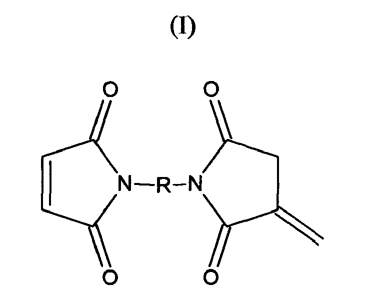

2. Composition according to Claim 1, R being chosen from the group consisting of alkylenes having from 1 to 20 carbon atoms, cycloalkylenes having from 6 to 24 carbon atoms, arylenes having from 6 to 18 carbon atoms and aralkylenes having from 7 to 25 carbon atoms.

3. Composition according to Claim 2, the itaconimidomaleimide being chosen from the group consisting of N-(itaconimido-m-phenyl)maleimide, N-(itaconimido-p-phenyl)maleimide, N-(itaconimido-o-phenyl)maleimide, N-(3-itaconimido-4,6-dimethylphenyl)maleimide, N-(3-itaconimido-4-methylphenyl)maleimide, N-(3-itaconimido-6-methylphenyl)maleimide, N-(3-itaconimido-2-methylphenyl)maleimide, N-(1'-itaconimido-4,4'-methylenebiphenyl)maleimide, N-[2-(methyleneitaconimido)phenyl]methylenemaleimide, N-[3-(methyleneitaconimido)phenyl]methylenemaleimide, N-[4-(methyleneitaconimido)phenyl]methylenemaleimide, and the mixtures of these compounds.

4. Composition according to Claim 2 or Claim 3, R being a phenylene group.

5. Composition according to Claim 4, the itaconimidomaleimide being N-(p-itaconimidophenyl)maleimide.

6. Composition according to any one of Claims 1 to 5, the diene elastomer being chosen from the group consisting of polybutadienes, natural rubber, synthetic polyisoprenes, butadiene copolymers, isoprene copolymers and the blends of these elastomers.

7. Composition according to any one of Claims 1 to 6, the reinforcing filler being present at a level of between 20 and 200 pce, preferably between 30 and 150 pce (parts by weight per hundred of diene elastomer).

8. Composition according to any one of Claims 1 to 7, the reinforcing filler being predominantly an inorganic filler, preferably silica.

9. Composition according to any one of Claims 1 to 7, the reinforcing filler being predominantly an organic filler, preferably carbon black.

10. Composition according to any one of Claims 6 to 9, the diene elastomer being an isoprene elastomer, preferably a synthetic polyisoprene, or a natural rubber.

11. Composition according to any one of Claims 1 to 10, the level of itaconimidomaleimide being between 0.1 and 10 pce.

12. Composition according to Claim 11, the level of itaconimidomaleimide being within a range from 0.2 to 2.5 pce.

13. Composition according to any one of Claims 1 to 12, **characterized in that** it additionally comprises a primary vulcanization accelerator preferably chosen from the group consisting of 2-mercaptobenzothiazyl disulphide, N-cyclohexyl-2-benzothiazolesulphenamide, N,N-dicyclohexyl-2-benzothiazolesulphenamide, N-tert-butyl-2-benzothiazolesulphenamide, N-tert-butyl-2-benzothiazolesulphenimide and the mixtures of these compounds.

14. Process for preparing a rubber composition which can be used for the manufacture of tyres and which exhibits an improved resistance to reversion, this composition being based on a diene elastomer, on a reinforcing filler and on a vulcanization system, the said process comprising the following stages:
• incorporating at least one reinforcing filler in a diene elastomer during a first "non-productive" stage, the combined mixture being kneaded thermomechanically, in one or more goes, until a maximum temperature of between 110°C and 190°C is reached;
• cooling the combined mixture to a temperature of less than 100°C;
• subsequently incorporating the vulcanization system during a second "productive" stage;
• kneading the combined mixture up to a maximum temperature of less than 110°C,
**characterized in that** an itaconimidomaleimide compound of formula (I) (R hydrocarbon radical comprising from 1 to 25 carbon atoms and optionally one or more heteroatom(s) chosen from O, N and S) : 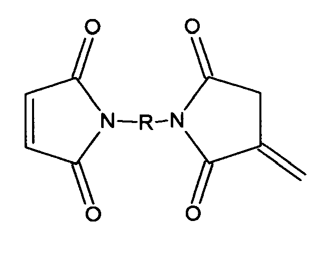 is additionally incorporated during any one of the stages of the process.

15. Process according to Claim 14, R being chosen from the group consisting of alkylenes having from 1 to 20 carbon atoms, cycloalkylenes having from 6 to 24 carbon atoms, arylenes having from 6 to 18 carbon atoms and aralkylenes having from 7 to 25 carbon atoms.

16. Process according to Claim 15, the itaconimidomaleimide being chosen from the group consisting of N-(itaconimido-m-phenyl)maleimide, N-(itaconimido-p-phenyl)maleimide, N-(itaconimido-o-phenyl)maleimide, N-(3-itaconimido-4,6-dimethylphenyl)maleimide, N-(3-itaconimido-4-methylphenyl)maleimide, N-(3-itaconimido-6-methylphenyl)maleimide, N-(3-itaconimido-2-methylphenyl)maleimide, N-(1'-itaconimido-4,4'-methylenebiphenyl)maleimide, N-[2-(methyleneitaconimido)phenyl]methylenemaleimide, N-[3-(methyleneitaconimido)phenyl]methylenemaleimide, N-[4-(methyleneitaconimido)phenyl]methylenemaleimide, and the mixtures of these compounds.

17. Process according to Claim 15 or Claim 16, R being a phenylene group.

18. Process according to Claim 17, the itaconimidomaleimide being N-(p-itaconimidophenyl)maleimide.

19. Process according to any one of Claims 14 to 18, the diene elastomer being chosen from the group consisting of polybutadienes, natural rubber, synthetic polyisoprenes, butadiene copolymers, isoprene copolymers and the blends of these elastomers.

20. Process according to any one of Claims 14 to 18, the reinforcing filler being present at a level of between 20 and 200 pce, preferably between 30 and 150 pce (parts by weight per hundred of diene elastomer).

21. Process according to any one of Claims 14 to 18, the reinforcing filler being predominantly an inorganic filler, preferably a silica.

22. Process according to any one of Claims 14 to 18, the reinforcing filler being predominantly an organic filler, preferably carbon black.

23. Process according to any one of Claims 19 to 22, the diene elastomer being an isoprene elastomer, preferably a synthetic polyisoprene, or natural rubber.

24. Process according to any one of Claims 14 to 23, the level of itaconimidomaleimide being between 0.1 and 10 pce.

25. Process according to Claim 24, the level of itaconimidomaleimide being within a range from 0.2 to 2.5 pce.

26. Process according to any one of Claims 14 to 25, the vulcanization system additionally comprising a primary vulcanization accelerator preferably chosen from the group consisting of 2-mercaptobenzothiazyl disulphide, N-cyclohexyl-2-benzothiazolesulphenamide, N,N-dicyclohexyl-2-benzothiazolesulphenamide, N-tert-butyl-2-benzothiazolesulphenamide, N-tert-butyl-2-benzothiazolesulphenimide and the mixtures of these compounds.

27. Use of a composition according to any one of Claims 1 to 13 for the manufacture of a finished article or of a semi-finished product intended for a motor vehicle ground-contact system.

28. Finished article or semi-finished product intended for a motor vehicle ground-contact system comprising a composition according to any one of Claims 1 to 13.

## Patentansprüche

1. Kautschukzusammensetzung, die zur Herstellung von Reifen verwendet werden kann, auf Basis von mindestens (i) einem Dienelastomer, (ii) einem verstärkenden Füllstoff, (iii) einem Vulkanisationssystem und (iv) einer Maleinimidverbindung, **dadurch gekennzeichnet, daß** es sich bei der Maleinimidverbindung um ein Itaconimidomaleinimid der Formel (R Kohlenwasserstoffrest mit 1 bis 25 Kohlenstoffatomen und gegebenenfalls einem oder mehreren unter O, N und S ausgewählten Heteroatomen): 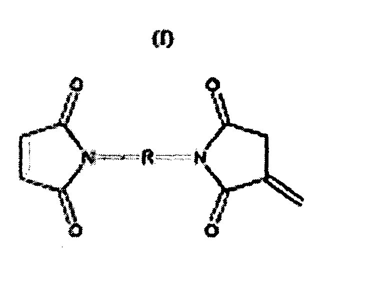 handelt.

2. Zusammensetzung nach Anspruch 1, wobei R aus der Gruppe bestehend aus Alkylenen mit 1 bis 20 Kohlenstoffatomen, Cycloalkylenen mit 6 bis 24 Kohlenstoffatomen, Arylenen mit 6 bis 18 Kohlenstoffatomen und Aralkylenen mit 7 bis 25 Kohlenstoffatomen ausgewählt ist.

3. Zusammensetzung nach Anspruch 2, wobei das Itaconimidomaleinimid aus der Gruppe bestehend aus N-(Itaconimido-m-phenyl)maleinimid, N-(Itaconimido-p-phenyl)maleinimid, N-(Itaconimido-o-phenyl)maleinimid, N-(3-Itaconimido-4,6-dimethylphenyl)maleinimid, N-(3-Itaconimido-4-methylphenyl)maleinimid, N-(3-Itaconimido-6-methylphenyl)maleinimid, N-(3-Itaconimido-2-methylphenyl)maleinimid, N-(1'-Itaconimido-4,4'-methylenbiphenyl)maleinimid, N-[2-(Methylenitaconimido)phenyl]methylenmaleinimid, N-[3-(Methylenitaconimido)phenyl]methylenmaleinimid, N-[4-(Methylenitaconimido)phenyl]methylenmaleinimid und Mischungen dieser Verbindungen ausgewählt ist.

4. Zusammensetzung nach Anspruch 2 oder 3, wobei R für eine Phenylengruppe steht.

5. Zusammensetzung nach Anspruch 4, wobei es sich bei dem Itaconimidomaleinimid um N-(p-Itaconimidophenyl)maleinimid handelt.

6. Zusammensetzung nach einem der Ansprüche 1 bis 5, wobei das Dienelastomer aus der Gruppe bestehend aus Polybutadienen, Naturkautschuk, synthetischen Polyisoprenen, Butadien-Copolymeren, Isopren-Copolymeren und Mischungen dieser Elastomere ausgewählt ist.

7. Zusammensetzung nach einem der Ansprüche 1 bis 6, wobei der verstärkende Füllstoff in einem Gehalt zwischen 20 und 200 pce und vorzugsweise zwischen 30 und 150 pce (Gewichtsteile pro hundert Teile Dienelastomer) vorliegt.

8. Zusammensetzung nach einem der Ansprüche 1 bis 7, wobei es sich bei dem verstärkenden Füllstoff hauptsächlich um einen anorganischen Füllstoff, vorzugsweise Siliciumdioxid, handelt.

9. Zusammensetzung nach einem der Ansprüche 1 bis 7, wobei es sich bei dem verstärkenden Füllstoff hauptsächlich um einen organischen Füllstoff, vorzugsweise Ruß, handelt.

10. Zusammensetzung nach einem der Ansprüche 6 bis 9, wobei es sich bei dem Dienelastomer um ein Isopren-Elastomer, vorzugsweise ein synthetisches Polyisopren oder Naturkautschuk, handelt.

11. Zusammensetzung nach einem der Ansprüche 1 bis 10, wobei der Gehalt an Itaconimidomaleinimid zwischen 0,1 und 10 pce liegt.

12. Zusammensetzung nach Anspruch 11, wobei der Gehalt an Itaconimidomaleinimid in einem Bereich von 0,2 bis 2,5 pce liegt.

13. Zusammensetzung nach einem der Ansprüche 1 bis 12, **dadurch gekennzeichnet, daß** sie außerdem einen primären Vulkanisationsbeschleuniger enthält, der vorzugsweise aus der Gruppe bestehend aus 2-Mercaptobenzothiazyldisulfid, N-Cyclohexyl-2-benzothiazylsulfenamid, N,N-Dicyclohexyl-2-benzothiazylsulfenamid, N-tert.-Butyl-2-benzothiazylsulfenamid, N-tert.-Butyl-2-benzothiazylsulfenimid und Mischungen dieser Verbindungen ausgewählt ist.

14. Verfahren zur Herstellung einer Kautschukzusammensetzung, die zur Herstellung von Reifen verwendet werden kann und eine verbesserte Reversionsbeständigkeit aufweist, wobei diese Zusammensetzung auf einem Dienelastomer, einem verstärkenden Füllstoff und einem Vulkanisationssystem basiert, bei dem man:
• im Lauf eines ersten, als "nichtproduktiv" bezeichneten Schritts mindestens einen verstärkenden Füllstoff in ein Dienelastomer einarbeitet, indem man das Ganze ein- oder mehrmals thermomechanisch knetet, bis eine Höchsttemperatur zwischen 110°C und 190°C erreicht ist;
• das Ganze auf eine Temperatur von weniger als 100°C abkühlt;
• dann im Lauf eines zweiten, als "produktiv" bezeichneten Schritts das Vulkanisationssystem einarbeitet;
• das Ganze bis zu einer Höchsttemperatur von weniger als 110°C knetet,
**dadurch gekennzeichnet, daß** man außerdem im Lauf eines der Verfahrensschritte eine Itaconimidomaleinimidverbindung der Formel (I) (R Kohlenwasserstoffrest mit 1 bis 25 Kohlenstoffatomen und gegebenenfalls einem oder mehreren unter O, N und S ausgewählten Heteroatomen): 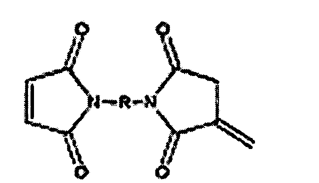 einarbeitet.

15. Verfahren nach Anspruch 14, wobei R aus der Gruppe bestehend aus Alkylenen mit 1 bis 20 Kohlenstoffatomen, Cycloalkylenen mit 6 bis 24 Kohlenstoffatomen, Arylenen mit 6 bis 18 Kohlenstoffatomen und Aralkylenen mit 7 bis 25 Kohlenstoffatomen ausgewählt ist.

16. Verfahren nach Anspruch 15, wobei das Itaconimidomaleinimid aus der Gruppe bestehend aus N-(Itaconimido-m-phenyl)maleinimid, N-(Itaconimido-p-phenyl)maleinimid, N-(Itaconimido-o-phenyl)-maleinimid, N-(3-Itaconimido-4,6-dimethylphenyl)-maleinimid, N-(3-Itaconimido-4-methylphenyl)-maleinimid, N-(3-Itaconimido-6-methylphenyl)-maleinimid, N-(3-Itaconimido-2-methylphenyl)-maleinimid, N-(1'-Itaconimido-4,4'-methylenbiphenyl)maleinimid, N-[2-(Methylenitaconimido)-phenyl]methylenmaleinimid, N-[3-(Methylenitaconimido)phenyl]methylenmaleinimid, N-[4-(Methylenitaconimido)phenyl]methylenmaleinimid und Mischungen dieser Verbindungen ausgewählt ist.

17. Verfahren nach Anspruch 15 oder 16, wobei R für eine Phenylengruppe steht.

18. Verfahren nach Anspruch 17, wobei es sich bei dem Itaconimidomaleinimid um N-(p-Itaconimidophenyl)-maleinimid handelt.

19. Verfahren nach einem der Ansprüche 14 bis 18, wobei das Dienelastomer aus der Gruppe bestehend aus Polybutadienen, Naturkautschuk, synthetischen Polyisoprenen, Butadien-Copolymeren, Isopren-Copolymeren und Mischungen dieser Elastomere ausgewählt ist.

20. Verfahren nach einem der Ansprüche 14 bis 19, wobei der verstärkende Füllstoff in einem Gehalt zwischen 20 und 200 pce und vorzugsweise zwischen 30 und 150 pce (Gewichtsteile pro hundert Teile Dienelastomer) vorliegt.

21. Verfahren nach einem der Ansprüche 14 bis 20, wobei es sich bei dem verstärkenden Füllstoff hauptsächlich um einen anorganischen Füllstoff, vorzugsweise Siliciumdioxid, handelt.

22. Verfahren nach einem der Ansprüche 14 bis 20, wobei es sich bei dem verstärkenden Füllstoff hauptsächlich um einen organischen Füllstoff, vorzugsweise Ruß, handelt.

23. Verfahren nach einem der Ansprüche 19 bis 22, wobei es sich bei dem Dienelastomer um ein Isopren-Elastomer, vorzugsweise ein synthetisches Polyisopren oder Naturkautschuk, handelt.

24. Verfahren nach einem der Ansprüche 14 bis 23, wobei der Gehalt an Itaconimidomaleinimid zwischen 0,1 und 10 pce liegt.

25. Verfahren nach Anspruch 24, wobei der Gehalt an Itaconimidomaleinimid in einem Bereich von 0,2 bis 2,5 pce liegt.

26. Verfahren nach einem der Ansprüche 14 bis 25, wobei das Vulkanisationssystem außerdem einen primären Vulkanisationsbeschleuniger enthält, der vorzugsweise aus der Gruppe bestehend aus 2-Mercaptobenzothiazyldisulfid, N-Cyclohexyl-2-benzothiazylsulfenamid, N,N-Dicyclohexyl-2-benzothiazylsulfenamid, N-tert.-Butyl-2-benzothiazylsulfenamid, N-tert.-Butyl-2-benzothiazylsulfenimid und Mischungen dieser Verbindungen ausgewählt ist.

27. Verwendung einer Zusammensetzung nach einem der Ansprüche 1 bis 13 zur Herstellung eines Fertigartikels oder Halbzeugs, das für ein Motorfahrzeug-Bodenkontaktsystem vorgesehen ist.

28. Fertigartikel oder Halbzeug, das für ein Motorfahrzeug-Bodenkontaktsystem vorgesehen ist, umfassend eine Zusammensetzung nach einem der Ansprüche 1 bis 13.
